# EUROPÄISCHE PATENTSCHRIFT

(11) **EP 2 879 506 B1**
(45) Veröffentlichungstag und Bekanntmachung des Hinweises auf die Patenterteilung: **31.01.2018**
(21) Anmeldenummer: 13789703.9
(22) Anmeldetag: 24.10.2013
(51) Int. Cl.: A23D 7/00, A23D 7/01, A23D 7/05, A21D 2/16, A21D 8/06, A61K 9/107, A61K 8/06

(54) **LEBENSMITTELFETTSYSTEM ODER KOSMETIKFETTSYSTEM ODER PHARMAFETTSYSTEM**
FAT SYSTEM FOR USE IN FOODS, COSMETICS OR PHARMACEUTICALS
SYSTÈME DE MATIÈRE GRASSE ALIMENTAIRE OU SYSTÈME DE MATIÈRE GRASSE COSMÉTIQUE OU SYSTÈME DE MATIÈRE GRASSE PHARMACEUTIQUE

(30) Priorität: 29.10.2012 DE 102012021545
(43) Veröffentlichungstag der Anmeldung: 10.06.2015
(73) Patentinhaber: ETH Zürich, 8092 Zürich (CH); MIFA AG FRENKENDORF, 4402 Frenkendorf (CH); Florin AG, 4132 Muttenz (CH)
(72) Erfinder: WINDHAB, Erich J., 8261 Hemishofen (CH); SPITZBARTH, Heiko, 4332 Stein (CH); HANSELMANN, William, 9495 Triesen (LI); BAUMANN, René, 4600 Olten (CH); ZWANZIG, Dirk, 4055 Basel (CH)
(74) Vertreter: Beyer, Rudi
(86) Internationale Anmeldenummer: PCT/EP2013/003198
(87) Internationale Veröffentlichungsnummer: WO 2014/067637

(56) Entgegenhaltungen:
- EP-A2- 0 238 199
- WO-A1-2010/039036
- WO-A1-2012/041682
- JAHANIAVAL FIROUZ ET AL: "CHARACTERIZATION OF A DOUBLE EMULSION SYSTEM (OIL-IN-WATER-IN-OIL EMULSION) WITH LOW SOLID FATS: MICROSTRUCTURE", JOURNAL OF THE AMERICAN OIL CHEMISTS' SOCIETY, SPRINGER, DE, Bd. 80, Nr. 1, 1. Januar 2003 (2003-01-01) , Seiten 25-31, XP002277209, ISSN: 0003-021X, DOI: 10.1007/S11746-003-0645-9

## Beschreibung

Die Erfindung betrifft ein Fettsystem, z. B. ein Lebensmittelfettsystem.

Des Weiteren betrifft die Erfindung ein Fettsystem, z. B. ein Kosmetikfettsystem. Außerdem betrifft die Erfindung ein Fettsystem, z. B. ein Pharmafettsystem. Schließlich betrifft die Erfindung ein Produkt zur Verwendung in fetthaltigen Lebensmitteln, Kosmetika und Pharmaka.

Der Umfang der Erfindung wird durch die Patentansprüche bestimmt. All die Anwendungsbereiche gemäß der Erfindung, also sowohl die Lebensmittelfettsysteme, als auch die Kosmetikfettsysteme, die Pharmafettsysteme und die erfindungsgemäßen Produkte unterliegen einem einheitlichen Erfindungsgedanken, nämlich der Strukturierung/Substrukturierung von Multiphasen-Fettmassen, die über einen gewissen breiten Temperaturbereich weitestgehend temperaturunabhängiges Konsistenz- und Stabilitätsverhalten aufweisen sowie einstellbare, technofunktionelle und/oder ernährungsphysiologisch relevante Eigenschaften besitzen.

### Stand der Technik

Fette oder fetthaltige Massen können in vielfältigster Form in Lebensmittel-, Pharma- und Kosmetik-Bereichen eingesetzt werden. Dabei können sie direkt vom Konsumenten angewendet werden wie z.B. Butter, Margarine, Hautcremes und Salben oder als Halbfabrikate von der Industrie weiterverarbeitet werden wie z.B. Ziehmargarinen in der Blätterteigherstellung.

Im allgemeinen bestehen solche Fettsysteme meist aus einem Gemisch aus kristallisierten Fettanteilen und flüssigem Fett bei einer bestimmten Temperatur, z.B. Raumtemperatur. Dabei wird der kristallisierte Fettanteil als Hartfett oder strukturierendes Fett bezeichnet. Diese Hartfette können verschiedene Funktionen übernehmen. Sie haben einen direkten Einfluss auf das Fließverhalten des Endproduktes. Dies ist ein sehr wichtiger Qualitätsfaktor wenn es darum geht, eine Creme mit der Hand auf die Haut aufzutragen, ein Ziehfett in dünnen Schichten in einen Teig einzuarbeiten oder auch beim direkten Verzehr von solchen Fettmassen. Wichtige Faktoren eines Hartfetts sind Art und Schmelzpunkt des Fetts, Anteil im Gesamtfettsystem, Kristallgröße oder Aufbau eines Kristallnetzwerks. Je nach Produkt und Anwendung liegt der Hartfettanteil zwischen 20-80% w/w bezogen auf Gesamtfett.

Um das plastische Verhalten oder den Kaloriengehalt von solchen Fettmassen einzustellen, kann zusätzlich eine Wasserphase in die flüssige Fettphase (= Ölphase) dispergiert werden. Dabei entsteht eine w/o-Emulsion, deren Struktur zusätzlich durch die in der Ölphase suspendierten Hartfett-Kristallpartikeln stabilisiert werden kann.

Generell sind Fettsysteme hinsichtlich ihres Konsistenz- bzw. Fliessverhaltens sehr temperaturlabil. Je nach Temperatur können Fettfraktionen im Gesamtsystem auskristallisieren oder aufschmelzen. Dies kann zu sehr großen makrostrukturellen Schwankungen und einhergehender Änderung der Eigenschaften innerhalb eines Bereiches von wenigen Grad Celsius führen. Gerade beim Auftragen einer Creme auf die Hautoberfläche oder das Einarbeiten von Ziehfetten in einen Teig ist möglichst weitgehend konstantes plastisches Verhalten der Masse der entscheidende Qualitätsfaktor.

Hartfette besitzen in der Regel hohe Gehalte an gesättigten Fettsäuren und Trans-Fettsäuren. Neuere Forschungen belegen, dass solche Inhaltskomponenten einen negativen Einfluss auf die Herz-Kreislaufgesundheit haben. Ferner sind palmölbasierte Hartfette wegen der Regenwaldabholzung in Folge Errichtung von großen Palmplantagen in die öffentliche Kritik geraten. Ferner ist die Fettleibigkeit nicht nur in der industrialisierten Welt sondern auch in urbanen Regionen von Entwicklungsländern ein zunehmend ernsthaftes Gesundheitsproblem geworden. Darum ist die Kalorienreduktion bei Lebensmitteln ein wichtiger Verkaufsfaktor gerade bei Fettsystemen mit deren typischerweise hohen Energiedichte.

Alle bisherigen Strategien zur Kalorienreduktion bestanden darin, eine wässrige Phase in die kontinuierliche, Hartfettpartikeln beinhaltende fluide Ölphase in dispergierter Form einzubringen. In der WO 2010/069747 ist ein fettreduzierter Brotaufstrich (<40% Gesamtfett) mit nicht gelierenden Proteinen in der dispersen wässrigen Phase beschrieben. In der WO 2010/069752 ist diese wässrige Phase geliert, um einen fettreduzierten Brotaufstrich herzustellen (<45% Gesamtfett).

Solche Fettsysteme kann man auf vielfältige Weise produzieren. Die meistverbreitete Herstellungsmethode beinhaltet folgende Schritte:
1. Mischen des flüssigen Hartfetts mit der flüssigen Ölphase und falls vorhanden dem wässrigen Anteil, um eine Voremulsion herzustellen.
2. Kühlen dieser Voremulsion unter mechanischem Energieeintrag, um eine w/o-Emulsion herzustellen und das Hartfett auszukristallisieren.
3. Einstellen der nötigen Plastizität durch weitergehendes Feindispergieren der Wassertropfen und Hartfettkristalle z.B. in einem Pinmischer.
4. Aufbau eines Kristallnetzwerkes in einem kontrolliert-temperierten "Halterohr".
5. Ausformung und Verpackung des Produktes.

Der vorab beschriebene Prozess ist sehr energieintensiv und die Kristallisation des Hartfetts ist beeinflusst vom eutektischen Verhalten in der Mischung des Hartfettes mit dem flüssigen Öl. Zudem wird in Folge Energiedissipation durch den hohen mechanischen Energieeintrag Hartfett wieder aufgeschmolzen, sodass während Lagerung und/oder Transport das Hartfett strukturell unkontrolliert rekristallisiert. Dadurch wird die Plastizität des resultierenden Produktes massiv beeinflusst, was z.B. bei der Weiterverarbeitung in Blätterteigen zu großen Problemen führen kann.

In einem andern Herstellungsverfahren wird bereits auskristallisiertes Hartfett in Form eines Pulvers der flüssigen Ölphase beigemischt. Diese alternative Herstellungsmethode führt zu großen Energieeinsparungen. Zudem wird die Nachkristallisation während Lagerung und Vertrieb auf ein Minimum reduziert. Solch ein Fettpulver kann durch Kaltsprühen (EP 1 285 584), durch superkritische Schmelzmikronisation (WO 2010/069752) oder durch Phaseninversion (EP 0 293 980) hergestellt werden.

In der WO 2006/087090 wurden scheibenförmige Primärpartikel (Dicke 0.01-0.5 µm) mittels flüssigem Öl oder w/o-Emulsionen zu 0.5-10mm Granulaten agglomeriert. Durch Einmischen in flüssiges Öl brechen diese Granulate wieder in Primärpartikel auf, und entfalten dann ihre Wirkung als Strukturbildner. Der vorgeschaltete Agglomerationsschritt ist der besseren Handhabung des Fettpulvers zuträglich.

Aus der DE 197 50 479 A1 ist ein Verfahren zum Erzeugen von wasserhaltigen, gefrorenen bzw. erstarrten, lagerstabilen, rieselfähigen Pulvermikrokapseln vorbekannt.

Patentanspruch 11 beschreibt dabei ein Verfahren mit den Merkmalen des Anspruches 1, bei dem aufgrund Versprühen einer O/W/O-Emulsion in den fettigen/öligen Sprühtropfen enthaltene dispergierte Wassertröpfchen ihrerseits eine zweite, ebenfalls in Tröpfchenform feinstdispergierte Fett-/Ölphase aufweisen, wobei der Gesamttropfen bei Erstarrung der äußeren Fett-/Ölphase verfestigt ist und die innere zweite Fett-/Ölphase bei Lagertemperatur im Gegensatz zur äußeren Fett-/Ölphase im geschmolzenen bzw. teilgeschmolzenen Zustand vorliegen kann.

Die DE 697 36 679 T2 betrifft die Herstellung eines fließfähigen Fettes in bestimmten Gewichtsverhältnissen mit Füllstoffen, die sehr speziell sind.

Der Druckschrift JAHÄNIAVAL FIROUZ ET AL, "Characterization of a double emulsion System (Oil-in-Water-in-oil emulsiöh) with low solid fats: microstructure", Journal pf the American oil chemists' society, Spinger, DE (20030101), Vol. 80, No. 1, doi:10.1007/ S11746-003-0645-9, ISSN 0003-021 X, pages 25 -31, XP002277209 ist eine Emulsion mit einem hochschmelzenden Fett, einem niedrigschmelzenden Fett sowie Wasser zu entnehmen. Das hochschmelzende Fett wird in der Mischung aus niedrigschmelzenden Fett und Wasser suspendiert. Wasserblasen können auch in der Emulsion gefunden werden. Die Phasen werden getrennt durch Abkühlung der Emulsion erhalten.

### Aufgabe

Der Erfindung liegt die generelle Aufgabe zugrunde, Konsistenz-, Fliess- und Stabilitätsverhalten von Fettsystemen für Lebensmittel, Kosmetika und Pharmaerzeugnisse sowie die dafür benötigten Produkte besser anwendungsspezifisch einzustellen und neuartige Textur verbessernde Strukturbildungseigenschaften in derartige Produkte einzubringen.

Insbesondere liegt der Erfindung die Aufgabe zugrunde, Fettsysteme, z. B. Lebensmittelfettsysteme mit deutlich reduziert temperaturabhängigen Konsistenz-, Fliess- und Stabilitätsverhalten sowie bevorzugt kalorienreduzierte und funktionalisierte Fettsysteme für Lebensmittel zu schaffen, welche im Lebensmittel bei dessen Herstellungsprozess verbesserte sensorische und technofunktionelle Textureigenschaften erzeugen lassen.

Des Weiteren liegt der Erfindung die Aufgabe zugrunde, Kosmetikfettsysteme mit deutlich reduziert temperaturabhängigem Konsistenz-, Fliess- und Stabilitätsverhalten sowie bevorzugt kalorienreduzierte und funktionalisierte Fettsysteme mit verbesserten Texturbildungseigenschaften für Kosmetika zu schaffen.

Außerdem liegt der Erfindung die Aufgabe zugrunde, innerhalb der Gesamtaufgabe Pharmafettsysteme mit deutlich reduziert temperaturabhängigem Konsistenz-, Fliess- und Stabilitätsverhalten sowie bevorzugt funktionalisierte Fettsysteme für Pharmaprodukte mit verbesserten Texturbildungseigenschaften zu schaffen.

Schließlich liegt der Erfindung die Aufgabe zugrunde, ein Produkt zu schaffen, das deutlich reduziert temperaturabhängige Konsistenz-, Fliess- und Stabilitätseigenschaften aufweist und das für Lebensmittelfettsysteme, Kosmetikfettsysteme und für Pharmafettsysteme spezifisch funktionalisierbar ist.

Bei all diesen Fettsystemen wird gleichzeitig angestrebt, einerseits die Kaloriendichte zu senken, andererseits gezielt funktionelle Eigenschaften, bevorzugt in eine Hartfettphase einzuschließen. Dabei soll insbesondere der Gesamthartfettanteil gesenkt werden, ohne dessen strukturierende Eigenschaften im Fettsystem zu verlieren, wobei die Fettmasse über einen gewissen Temperaturbereich gleichbleibend fließfähig bzw. plastisch verformbar und stabil bleiben und neuartige Texturbildungseigenschaften in die resultierenden Endprodukte zur Verbesserung deren Herstellungs-, Verzehrs- und Applikationseigenschaften einbringen soll.

### Lösung der Aufgabe betreffend ein Lebensmittelfettsystem oder Kosmetikfettsystem oder Pharmafettsystem

Die Aufgabe wird durch die Merkmale des **Patentanspruchs 1** gelöst.

### Lösung der Aufgabe für ein Produkt

Die Aufgabe wird durch die in **Patentanspruch 22** wiedergegebenen Merkmale gelöst.

### Einige Vorteile

Die in allen Fettsystemen, also sowohl in Lebensmittelfettsystemen, Kosmetikfettsystemen, Pharmafettsystemen und bei den Produkten eingesetzte Multiphasen-Fettmassen verwenden unter anderem Hartfette, wobei sich die Bezeichnung auf ein Fett bezieht, welches bei Raumtemperatur von etwa 20° C fest ist.

Die vorliegende Erfindung bezieht sich auf die Substrukturierung von Hartfetten in Partikelform mit innerer Substruktur in Fettmassen, die durch (a) verbessert einstellbare Strukturstabilität, Konsistenz und plastische Fliesseigenschaften über einen breiteren Temperaturbereich, (b) eine Reduktion der Kaloriendichte (c) die Erzeugung neuartiger sensorisch und technofunktionell relevanter Texturmerkmale sowie (d) durch vereinfachte Funktionalisierbarkeit mit nutritiven und/oder kosmetischen und/oder pharmazeutischen Stoffkomponenten gekennzeichnet ist.

Durch den herkömmlichen Prozess ist es nicht möglich, das Hartfett hinreichend definiert zu strukturieren, da das Hartfett am Anfang der Herstellung ebenfalls in flüssiger Form in der Ölphase vermischt ist und in aller Regel in Folge Mischbarkeit bzw. Teilmischbarkeit von Hartfett und Oel Fraktionen eutektikale Effekte Einfluss auf den Schmelztemperaturbereich (z.B. Schmelztemperaturabsenkung) der auskristalli-sierenden Hartfettfraktionen haben. Ferner schließt die herkömmliche Herstellung von Hartfettfraktionen die Einbindung von z.B. Wasser oder Luft-/Gasanteilen in dieselben aus.

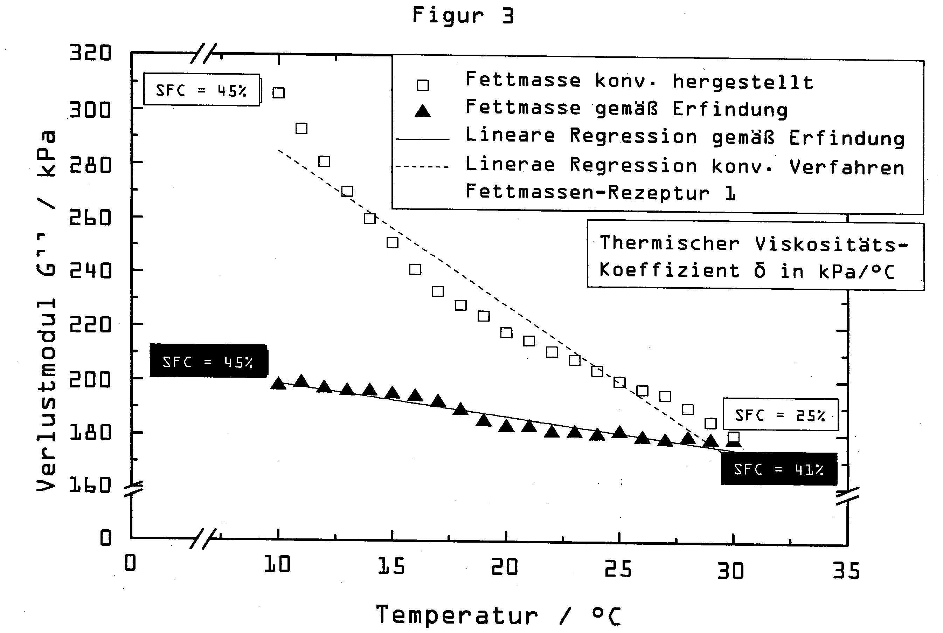

Durch die erfindungsgemäße Substrukturierung kann das Hartfett ohne Wechselwirkungseffekte mit der Oelphase maßgeschneidert strukturiert und Wasser sowie Luft-/Gasanteile eingebunden werden, welche die Verarbeitungs- und Applikationseigenschaften durch neuartige Texturbildung maßgeblich verbessern lassen. Darüber hinaus erlauben die erfindungsgemäß substrukturierten Hartfettpartikeln die effizientere und vereinfachte Inkorporierung/Enkapsulierung funktioneller Stoffkomponenten, welche aus sensorischen, ernährungsphysiologischen und/oder medizinischen Gründen relevant sind. Darüber hinaus kann in Folge der Substrukturierung der Hartfettanteil oder generell der Fettanteil in den Endprodukten deutlich reduziert werden ohne die maßgeschneiderten Schmelz-, Konsistenz-, Fliess-, Stabilitäts- und neuartigen Texturbildungseigenschaften des Fettsystems negativ zu beeinflussen - Figur 3 der Zeichnung.

In den erfindungsgemäßen substrukturierten Hartfettfraktionen können hydrophobe und/oder hydrophile Inhaltsstoffe inkorporiert bzw. enkapsuliert werden, die durch eine feste, kristalline Hartfettumhüllung z.B. besser vor Diffusionsverlusten geschützt sind. Weiter zeigen die so hergestellten Fettmassen eine verbesserte Strukturstabilität über einen größeren Temperaturbereich. Dies ist z.B. bei der Verarbeitung von Ziehfetten in Teigen sehr wichtig, da durch die größere Strukturstabilität über einen erweiterten Temperaturbereich auch eine verbesserte Produktionsstabilität und somit weniger Fehlproduktion realisiert werden kann. Ein weiteres Beispiel aus dem Kosmetikbereich betrifft das Aufstreichverhalten einer Creme auf die Hautoberfläche, welches über einen, für den Konsumenten relevanten Temperaturbereich konstant gehalten wird.

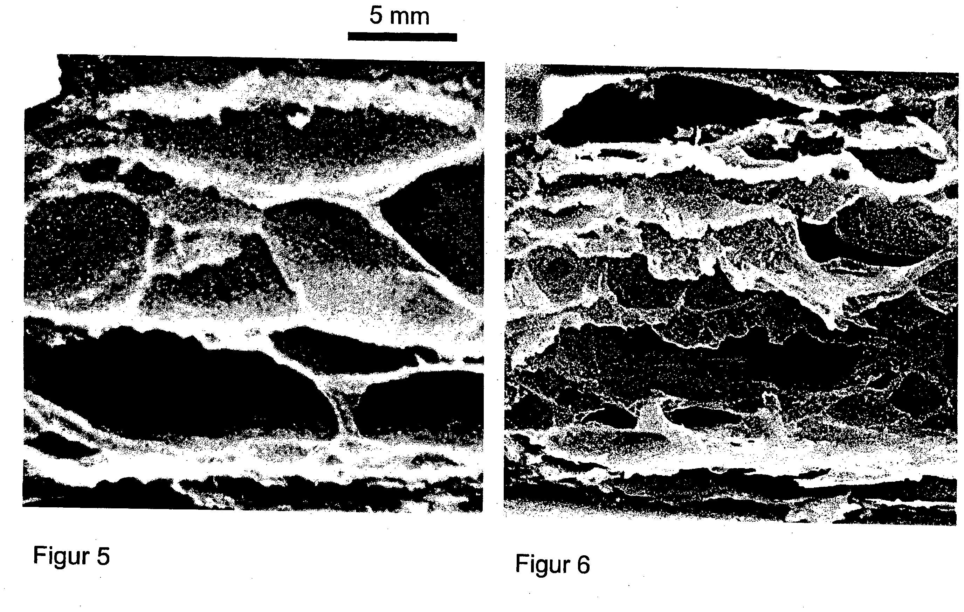

Neue texturverbessernde Eigenschaften gemäß der Erfindung resultieren zum Beispiel aus einer Inkorporation disperser Wassertröpfchen in die Hartfettpartikeln eines erfindungsgemäßen mikrostrukturierten Lebensmittel Composit-Fettsystems, das als Ziehfett bzw. Ziehmargarine bevorzugten Einsatz findet. Konzentration und Größe dieser inorporierten Wassertröpfchen sowie der Schmelztemperaturbereich der Hartfettpartikeln lassen die Triebkraft der Ziehmargarine im Backprozess regulieren. Damit kann der beim Backen entstehende Wasserdampf als Triebmittel in der fettbasierten Trennschicht gezielt an die Teigstrukturbildung im Backprozess, das heißt an die Verkleisterungstemper/-kinetik und Teigkrumenverfestigungstem-peratur/-kinetik angepasst werden. Damit werden Triebkraft und resultierendes Backvolumen optimiert und eine feinere Blättrigkeit der Teigstruktur im Backprodukt erzeugt (siehe beispielhafte Figuren 5 und 6).

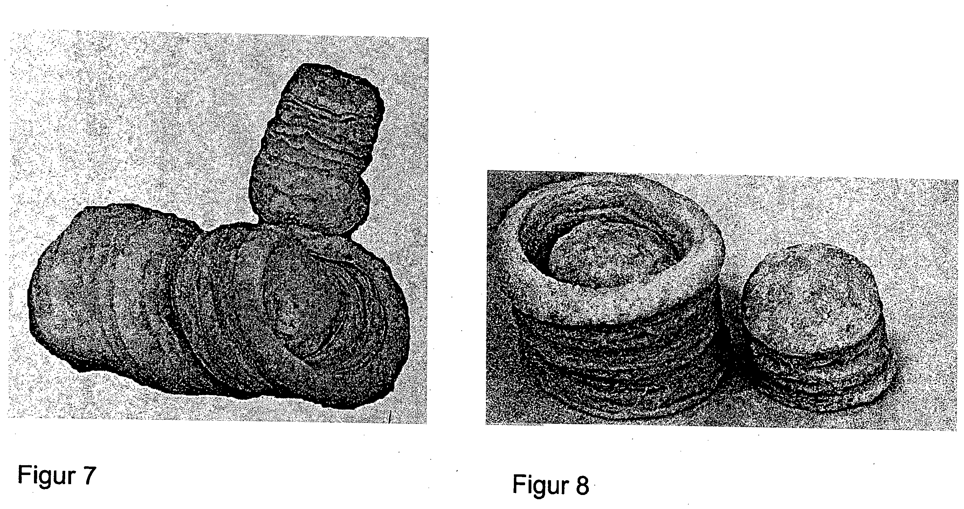

Des Weiteren ist es mit einem Ziehfett, hergestellt gemäß Erfindung möglich, Blätterteig herzustellen, welcher im Gegensatz zu herkömmlichen Blätterteigen auch in der Mikrowelle backfähig ist. Dieser Vorteil gegenüber konventionellen Produkten ist wiederum darauf zurückzuführen, dass die disperse Wasserphase, welche in den Hartfettpartikeln als substrukturierende Phase eingebracht ist, über Wasserkonzentration, Wassertropfengrößenverteilung und Hartfett-Schmelztemperaturbereich gezielt an die Backbedingungen in der Mikrowelle angepasst werden kann. Das Resultat ist ein mit einem Backofenprodukt vergleichbarer Blätterteig. Im Gegensatz dazu führt bei konventionell hergestelltem Blätterteig (mit konventionellem Ziehfett oder Ziehmargarine) das neuartige rationelle Backen in der Mikrowelle zu höchst unansehnlichen Produktstrukturen (siehe beispielhafte Figuren 7 und 8).

### Substrukturierung des Hartfettpulvers

Die Substrukturierung des Hartfetts kann durch ein Kaltsprühverfahren erfolgen, ist aber darauf nicht beschränkt. Dabei werden eine fettbasierende Suspension, Emulsion oder Schaum, bestehend aus der flüssigen Hartfettkomponente und den darin dispergierten (i) Feststoff(en), (ii) wässrigen Phase(n), oder (iii) Gasphasen, über eine Düse in eine kalte Gasphase versprüht. Dabei kristallisiert das flüssige Hartfett aus und schließt die darin dispergierten Phasen ein. Makroskopisch entsteht ein rieselfähiges Hartfett-Pulver. Das Hartfett hat den über die Fettkomposition eingestellten Schmelztemperaturbereich und die gekoppelten Verformungs-/Konsis-tenz und Fliesseigenschaften.

In die Hartfettphase können Biopolymere wie z.B. essbare Proteine oder Polysaccharide, einschließlich z.B. unverdaubaren Zellulosen, eingeschlossen werden. Zusätzlich können weitere, Inhaltsstoffe in die Hartfettphase eindispergiert werden. Ohne den Anspruch auf Vollständigkeit zu erheben, können dies z.B. Vitamine, Mineralien, Aromen, Alkohol, Kakao, Frucht-, Gemüse-, Nuss-, Fleisch- oder Fischstückchen und oder -pürees sein. Ferner können Emulgatoren für die Substrukturierung des Hartfetts eingesetzt werden. Der Anteil an nicht-Hartfettmaterial im gesamten Hartfett-Sprühpulverprodukt kann zwischen 0.01% und 70% liegen. Makroskopisch liegt ein rieselfähiges Pulver im Anwendungstempera-turbereich vor.

In einer anderen Ausführungsform des Erfindungsgegenstandes kann die Hartfettphase auch mit einer weiteren flüssigen Phase substrukturiert werden. Dabei wird diese flüssige, wässrige Phase in das flüssige Hartfett dispergiert / emulgiert und anschließend kaltgesprüht. Die wässrige Phase kann dabei z.B. aus Wasser, Milch, Frucht- oder Gemüsesäften, Kaffee- oder Teeextrakt bestehen. Ferner kann die wässrige Phase durch eine Gas- oder Fettphase oder feste Partikel weitergehend substrukturiert werden. Dabei werden geeignete Emulgatoren eingesetzt. Der Anteil an nicht-Hartfettmaterial kann wiederum zwischen 0.01% und 70% betragen. Makroskopisch entsteht ein rieselfähiges Hartfett-Sprühpulverprodukt.

In einer anderen Ausführung des Erfindungsgegenstandes kann die Hartfettphase durch ein Gas substrukturiert werden. Dabei wird ein Gas in die flüssige Hartfettphase eindispergiert und anschließend diese Dispersion kaltgesprüht. Das Verhältnis Gas:Hartfett kann zwischen 0.1 und 3:1 variieren. Makroskopisch entsteht wiederum ein rieselfähiges Hartfett-Pulver.

Im Allgemeinen besitzt ein so substrukturiertes Hartfettpulver runde Partikeln in einem über die Sprühparameter einstellbaren Durchmesserbereich von 0.1 - 500 µm. Die Partikelmorphologie kann aber auch andere geometrische Formen aufweisen, wie z.B. Ellipsoid-, Plättchen- oder unregelmäßige Formen.

### BEISPIEL

### Ziehfett mit 50% (w/w) Wassergehalt

Das Hartfett wurde aus den in Tabelle 1 beschriebenen Komponenten generiert und bei 80°C verflüssigt.

**Tabelle 1: beispielhafte Rezeptur für die kontinuierliche Hartfettphase zur Herstellung eines typischen substrukturierten Hartfettpulvers.**

| Hartfett | Schmelzpunkt [°C] | Gehalt [%] |
|---|---|---|
| Verestertes Palmfett (TFA frei) | 47-50 | 72 |
| Palmöl (unhydrogeniert) | 41-44 | 16 |
| Palmstearin | 61-63 | 12 |

5 % (w/w) Polyglycerin-polyricinoleat (PGPR) wurde als Emulgator zugegeben. Diese Hartfett-Komponentenmischung wurde in einem Rotor/Stator Mischer bei 6000 rpm für 2 Minuten gemischt und danach unter weiterem Mischen kontinuierlich 40 % (w/w) Wasser eindispergiert. Nach Zugabe des Wassers wurde die Emulsion für weitere 15 Minuten gemischt/dispergiert. Danach wurde diese Emulsion über eine Sprühdüse kaltversprüht (14 l/h). Die Lufttemperatur betrug -20°C und wurde über die Verdampfung von flüssigem Stickstoff eingestellt. Das so substrukturierte Hartfettpulver wurde am Fuß des Kaltsprühturms aus dem Prozessraum ausgebracht.

Das substrukturierte Hartfett wurde dann in eine w/o-Emulsion eindispergiert. Die kontinuierliche Phase der Emulsion bestand aus folgenden Inhaltskomponenten (Tabelle 2).

**Tabelle 2: Rezeptur für die kontinuierliche w/o-Emulsion**

| Gehalt [%] | Gehalt [%] |
|---|---|
| Rapsöl | 20 |
| Wasser | 80 |

Die Emulsion wurde mit 5% (w/w) PGPR stabilisiert. Zur Herstellung dieser Emulsion wurde Wasser kontinuierlich der Rapsölphase mit einem Rotor/Statormischer bei 6000 rpm zugemischt und für 10 Minuten weitergerührt.

Danach wurde das substrukturierte Hartfettsystem in diese w/o-Emulsion mittels Rühren bei 25°C im Verhältnis 3:1 eindispergiert.

Erfindungsgemäß stellt man durch Kaltsprühen einer fettbasierten Suspension, Emulsion oder Schaum ein substrukturiertes Fettpulver her. Dieses feste Pulver wird dann in einem weiteren erfindungsgemäßen Schritt in ein flüssiges Öl, eine w/o-Emulsion, fettbasierte Suspension oder einen fettbasierten Schaum eindispergiert. Dabei entsteht eine Fettmasse, die mechanisch stabil über den eingestellten Temperaturbereich für die disperse, gegebenenfalls substrukturierte Hartfett-Pulver Komponente ist, einen reduzierten Hartfettanteil aufweist sowie eine Mehrzahl an Möglichkeiten für die Funktionalisierung durch Einbindung von funktionellen Komponenten in die diversen dispersen Subphasen bietet.

In den Anmeldungsunterlagen werden die folgenden Begriffe wie folgt benutzt:
**a) Wasser-Öl-Emulsion**
   W/O-Emulsion = Wasser in Öl Emulsion = Wassertropfen als disperse Phase in kontinuierlicher Ölphase
**b) O/W/O Doppelemulsion**
   Öl in Wasser in Öl Emulsion = (kleinere) Wassertropfen in (größeren) Öltropfen in kontinuierlicher die Öltropfen umgebender Wasserphase
**c)** In aller Regel wird als Öl eine fluide Fettphase bezeichnet, welche bei Raumtemperatur (20 °C) komplett flüssig ist. Im Gegensatz dazu wird bei höherer Temperatur als Raumtemperatur ein schmelzendes Fett und deren fluider aufgeschmolzener Zustand nicht als Öl, sondern eben als (aufgeschmolzene) Fettphase bezeichnet.
**d) Öl-/Fettphase**
   Dies bedeutet Öl- oder Fettphase.
**e) Gas-/Luftblasen**
   Wird mit "oder" übersetzt.

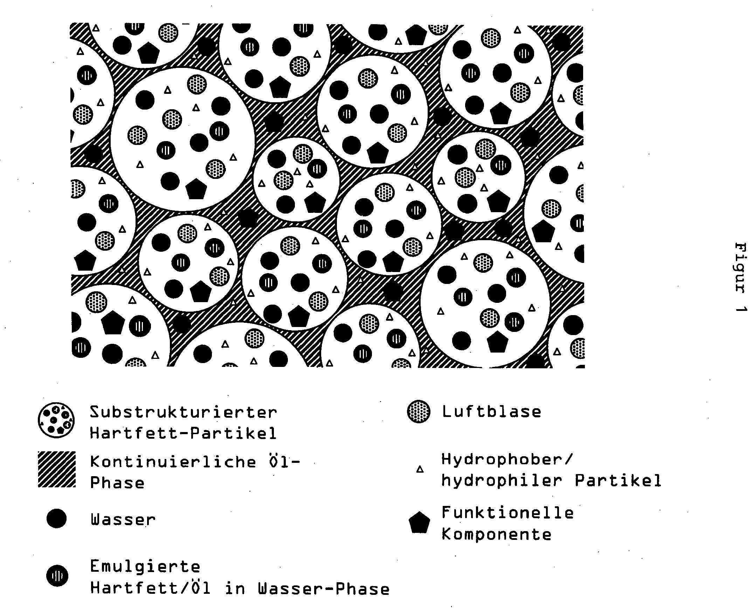

In den nachfolgend beschriebenen Figuren 1 - 8 ist die Erfindung ergänzend veranschaulicht:
Figur 1 zeigt einen schematischen Aufbau eines Fettsystems gemäß der Erfindung. Dabei sind substrukturierte Hartfettpartikel in flüssiger Ölphase dispergiert. Die Substrukturierung kann durch eine wässrige Phase, eine emulgierte o/w-Phase ein Gas, hydrophile oder hydrophobe Partikel oder funktionelle Komponenten erfolgen;

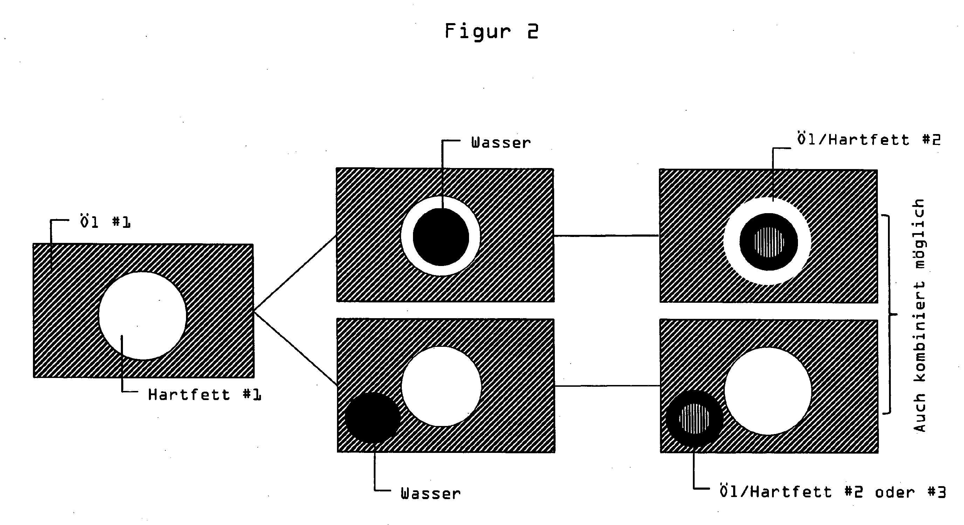

Figur. 2 zeigt die möglichen Kombinationsmöglichkeiten aus Hartfettpartikel, deren Substrukturierung durch Wasser und/oder o/w-Emulsion, in Öl und/oder w/o-Emulsion;
Figur 3 zeigt das Verlustmodul G" über der Temperatur für eine konventionell hergestellte Fettmasse und eine Fettmasse gemäß der Erfindung. Dabei verhält sich die Fettmasse gemäß der Erfindung über den Temperaturbereich von 10° bis 30°C nahezu stabil. Dagegen kann für eine konventionell hergestellte Fettmasse ein steiler Abfall der Struktur in demselben Temperaturbereich festgestellt werden. Der Verlust an Struktur spiegelt sich auch im Festfettgehalt (SFC) der beiden Massen wider. Für die Analyse des Verlustmoduls wurde ein Rotationsrheometer mit einer profilierten Platte-Platte Messgeometrie verwendet. Die Analyseparameter wurden folgendermaßen gewählt:

| | |
|---|---|
| Amplitude [%] | 0,1 |
| Kreisfrequenz ω [rad/s] | 1 |
| Temperaturrampe [°C], 1°C/min | 10 - 30 |
| Messpunktdauer [min] | 1 |

Die Messungen der Festfettgehalte der konventionell hergestellten Fettmasse wurde entsprechend der direkten AOCS-Methode Cd 16b-93 durchgeführt mittels Kernspinresonanz (NMR). Die Messung der Festfettgehalte der Fettmassen gemäß Erfindung wurde ebenfalls mittels Kernspinresonanz bei den jeweiligen Temperaturen von 10° C und 30° C durchgeführt. Die Steigungen der Regressionsgeraden entsprechen den jeweiligen Strukturerweichungsraten der dargestellten Fettmassen aufgrund einer Erwärmung von 10° C auf 30° C. Steigung für konventionell hergestellte Fettmasse: -5,7 kPa/C Steigung für mit neuem Verfahren hergestellte Fettmassen: -1,2 kPa/C SFC = Festfettgehalt in % (solid fat content) der jeweiligen Fettmassen bei Anfangstemperatur (10° C) und Endtemperatur (30° C);

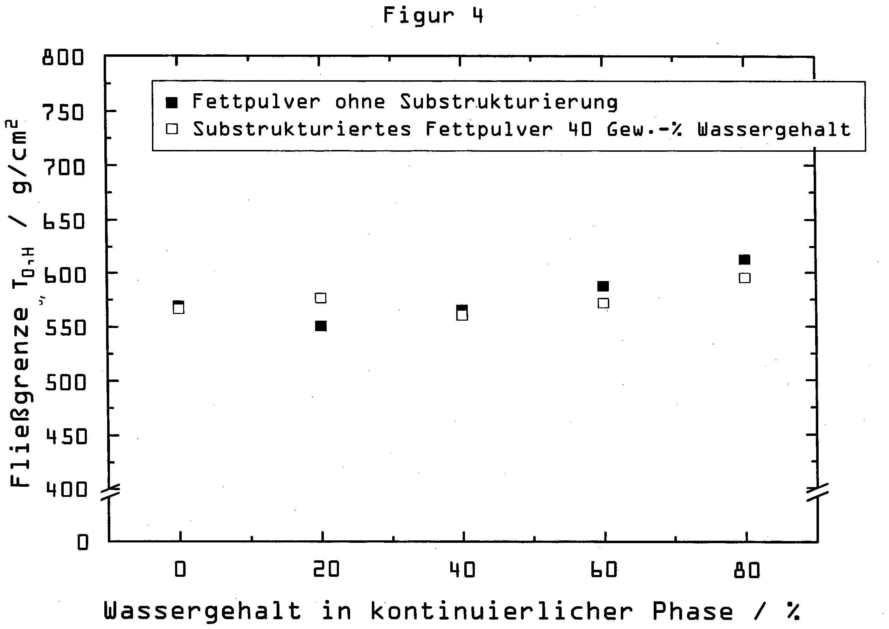

Figur 4 zeigt die Fließgrenze von 2 Fettmassen über dem Wassergehalt in der kontinuierlichen Phase. Dabei wurde in einem Fall das Hartfett mit einer wässrigen Phase (40% w/w) substrukturiert und im andern Fall nicht. Für beide Fettmassen liegen die Fließgrenzen eng zusammen. Dadurch sind die Strukturen sehr ähnlich, obwohl in einem Fall 40 % des Hartfetts eingespart werden konnte. Ergebnisse aus der Konuspenetration durchgeführt mit einem 30 Konus (Kegelwinkel 30 Grad). Diese Abbildung zeigt die Fließgrenze nach Haighton verschiedener Fettmischungen mit unterschiedlichen Wassergehalten in der kontinuierlichen Phase bzw. mit dispersen substrukturierten Partikeln. Die gefüllten Punkte zeigen die Ergebnisse mit Fettpulver ohne Wasserzusatz und die ungefüllten Punkte zeigen die Ergebnisse mit substrukturiertem Fettpulver mit 40 % Gew.% Wasseranteil.
Figur 5 zeigt die Zellstruktur eines Blätterteiges, hergestellt unter Verwendung eines konventionellen Ziehfettes;
Figur 6 zeigt die deutlich feinere Zellstruktur eines Blätterteiges (im Vergleich zu Figur 5), hergestellt unter Verwendung eines erfindungsgemäß substrukturierten Ziehfettes, welches disperse Wassertröpfchen inkorporiert in die Hartfettpartikeln enthält;
Figur 7 Blätterteig-Pastetenform mit konventionell hergestelltem Ziehfett in der Mikrowelle gebacken;
Figur 8 Blätterteig-Pastetenform mit erfindungsgemäß substrukturiert hergestelltem Ziehfett (Hartfettpartikeln mit incorporierten dospersen Wassertröpfchen) in der Mikrowelle gebacken.

### Literaturverzeichnis

DE 197 50 479 A1
DE 697 36 679 T2
WO 2006/087090
WO 2010/069747
WO 2010/069752
EP 1 285 584
EP 0 293 980

## Patentansprüche

1. Lebensmittelfettsystem oder Kosmetikfettsystem oder Pharmafettsystem mit deutlich reduziert temperaturabhängigen Konsistenz- und Stabilitätsverhalten sowie mit einstellbaren, technologisch und/oder ernährungsphysiologisch relevanten Funktionseigenschaften, wobei hochschmelzende, substrukturierte Fettpartikel in einer niedrigschmelzenden Fettphase oder Ölphase oder Wasser/Öl-Emulsion suspendiert sind, wobei durch die Trennung in eine niedrig- und eine hochschmelzende Ölfraktion oder Fettfraktion sowie die Anordnung der hochschmelzenden Hartfettphase in Form suspendierter disperser Hartfettpartikel in der niedrigschmelzenden Ölfraktionsphase sowie die zusätzliche Substrukturierung der dispersen Hartfettpartikel durch inkorporierte disperse Wassertropfen und/oder Luftblasen oder Gasblasen die Temperaturabhängigkeit der Viskosität des Gesamtsystems in einem Temperaturbereich von 10 bis 20 Grad Celsius, bevorzugt von 5 bis 30 Grad Celsius, weitergehend bevorzugt von 0 bis 40 Grad Celsius, quantitativ beschrieben durch den thermischen Viskositäts-Koeffizienten - Veränderung der Viskosität in kPas pro Grad Temperaturänderung in °C - um den Faktor > 3, bevorzugt um den Faktor > 5 oder > 6, reduziert einstellbar ist.

2. Fettsystem nach Anspruch 1, **dadurch gekennzeichnet, dass** der Schmelzpunkt der hochschmelzenden, dispersen, strukturierten Partikelphase > 20°C und der Erstarrungspunkt der niedrigschmelzenden Fett-/Ölphase < 15°C beträgt.

3. Fettsystem nach einem der Ansprüche 1 und 2, **dadurch gekennzeichnet, dass** der Schmelzpunkt der hochschmelzenden, dispersen, strukturierten Partikelphase > 40°C und der Erstarrungspunkt der niedrigschmelzenden Fett-/Ölphase < 5°C beträgt.

4. Fettsystem nach einem der Ansprüche 1 bis 3, **dadurch gekennzeichnet, dass** der Schmelzpunkt der hochschmelzenden, dispersen, strukturierten Partikelphase > 60°C und der Erstarrungspunkt der niedrigschmelzenden Fett-/Ölphase < 5°C, bevorzugt <0°C, beträgt.

5. Fettsystem nach Anspruch 1 oder einem der Ansprüche 2 bis 4, **dadurch gekennzeichnet, dass** die hochschmelzende, disperse, strukturierte Partikelfettphase eine erstarrte Emulsion mit inneren, stabilisierten Wassertropfen ist und/oder die kontinuierliche niedrigschmelzende Fett- oder Ölphase eine Wasser in Öl Emulsion mit stabilisierten Wassertropfen ist.

6. Fettsystem nach Anspruch 1 oder einem der Ansprüche 2 bis 5, **dadurch gekennzeichnet, dass** die hochschmelzende, disperse Partikelphase eine Öl-/Wasser/Öl-Doppelemulsion ist, deren äußere Ölphase (O) aus einem hochschmelzenden Fett mit Schmelztemperaturen > 20°C, bevorzugt > 40°C, insbesondere bevorzugt > 60°C, beträgt und/oder die kontinuierliche niedrigschmelzende Fett-/Ölphase eine Wasser/Öl-Emulsion oder Öl-/Wasser/Öl-Emulsion ist, mit einer äußeren kontinuierlichen Emulsions-Ölphase, welche Erstarrungstemperaturen < 15°C, vorzugsweise < 5°C, weitergehend bevorzugt < 0° C, aufweist.

7. Fettsystem nach Anspruch 1 oder einem der Ansprüche 2 bis 6, **dadurch gekennzeichnet, dass** die disperse Partikelstruktur in ihrer hochschmelzenden Fettphase und/oder der inneren Wasserphase und/oder der innersten Öl-/Fettphase Antioxidantien, poly-ungesättigte Fettsäuren und/oder andere ernährungsphysiologisch oder gesundheitsfördernd und/oder organoleptisch relevante funktionale Komponenten aufweist.

8. Fettsystem nach Anspruch 1 oder einem der Ansprüche 2 bis 7, **dadurch gekennzeichnet, dass** die kontinuierliche Fluidphase, welche einem niedrigschmelzenden Fett-/Ölsystem mit oder ohne beinhalteten Emulsions- bzw. Doppelemulsionssubstrukturen entspricht, Antioxidantien, poly-ungesättigte Fettsäuren und/oder andere ernährungsphysiologisch- oder gesundheitsfördernd und/oder organoleptisch relevante funktionale Komponenten und/oder kalorienarme Füllkomponenten in gelöster und/oder dispergierter Form inkorporiert, aufweist.

9. Fettsystem nach Anspruch 1 oder einem der Ansprüche 2 bis 8, **dadurch gekennzeichnet, dass** in einer oder mehreren der dispersen Fett-/Ölphasen und/oder Wasserphasen ernährungsphysiologisch- oder gesundheitsfördernd und/oder organoleptisch relevante funktionale Komponenten und/oder kalorienarme Füllkomponenten in gelöster und/oder dispergierter Form inkorporiert sind.

10. Fettsystem nach Anspruch 1 oder einem der Ansprüche 2 bis 9, **dadurch gekennzeichnet, dass** in die kontinuierliche Fett-/Ölphase und/oder eine oder mehreren der dispersen Fett-/Ölphasen und/oder Wasserphasen, Gas-/Luftblasen inkorporiert sind, welche in der jeweiligen umgebenden Phase eine Gasdispersions- oder Schaumstruktur ausbilden.

11. Fettsystem nach Anspruch 1 oder einem der Ansprüche 2 bis 10, **dadurch gekennzeichnet, dass** der Masseanteil der substrukturierten oder nicht substrukturierten hochschmelzenden Hartfettpartikel in der kontinuierlichen niedrigschmelzenden als w/o-Emulsion substrukturierten oder nicht derart substrukturierten Öl-/Fettphase, bezogen auf die Gesamtmasse, zwischen 5 % und 85 %, bevorzugt zwischen 10 % und 75 %, beträgt.

12. Fettsystem nach Anspruch 1 oder einem der Ansprüche 2 bis 11, **dadurch gekennzeichnet, dass** der Massenanteil in die Hartfettpartikel inkorporierter disperser Wassertropfen, bezogen auf die Hartfettmasse, zwischen 0 % und 80 % beträgt.

13. Fettsystem nach Anspruch 1 oder einem der Ansprüche 2 bis 12, **dadurch gekennzeichnet, dass** der Volumenanteil in die Hartfettpartikel inkorporierter disperser Gas-/Luftblässchen, bezogen auf das Hartfettvolumen, zwischen 0 % und 75 % beträgt.

14. Fettsystem nach Anspruch 1 oder einem der Ansprüche 2 bis 13, **dadurch gekennzeichnet, dass** der Massenanteil in die niedrigschmelzende kontinuierliche Öl-/Fettphase inkorporierter disperser Wassertropfen, bezogen auf die Gesamtmasse der kontinuierlichen Phase, zwischen 0 % und 80% beträgt.

15. Fettsystem nach Anspruch 1 oder einem der Ansprüche 2 bis 14, **dadurch gekennzeichnet, dass** die als Plastizitätsindikator mittels Kegel-Penetrationsmessung ermittelte Fliessgrenze τ_{0,H} des Gesamtfettsystems mit maximalen Abweichungen in Höhe +/- 5% vom gemessenen Mittelwert weitestgehend unabhängig ist vom Anteil des (i) in die Hartfettpartikel sowie (ii) in die kontinuierliche niedrigschmelzende Öl-/Fettphase durch Substrukturierung in Form disperser Wassertropfen integrierten Wasseranteils in einem Bereich desselben von 0 % bis 80%.

16. Fettsystem nach Anspruch 1 oder einem der Ansprüche 2 bis 15, **dadurch gekennzeichnet, dass** der Volumenanteil in die niedrigschmelzende kontinuierliche Öl-/Fettphase inkorporierter disperser Gas-/Luftblässchen, bezogen auf das Gesamtvolumen dieser kontinuierlichen Phase, zwischen 0 % und 75 % beträgt.

17. Fettsystem nach Anspruch 1 oder einem der Ansprüche 2 bis 16, **dadurch gekennzeichnet, dass** der Gesamtfettgehalt zwischen 20 % und 100 %, bevorzugt zwischen 50 % und 100 %, beträgt.

18. Fettsystem nach Anspruch 1 oder einem der Ansprüche 2 bis 17, **dadurch gekennzeichnet, dass** der Gesamtkaloriengehalt bei Substrukturierung der dispersen Hartfettpartikelphase durch Inkorporation disperser Wassertropfen und/oder Luft-/Gasblässchen um 50 %, bevorzugt um 60 %, reduziert ist.

19. Fettsystem nach Anspruch 1 oder einem der Ansprüche 2 bis 18, **dadurch gekennzeichnet, dass** der Gesamtkaloriengehalt bei Substrukturierung der dispersen Hartfettpartikelphase und der kontinuierlichen niedrigschmelzenden Öl-/Fettphase durch Inkorporation disperser Wassertropfen und/oder Luft-/Gasblässchen um > 50 %, bevorzugt um > 70 %, reduziert ist.

20. Fettsystem nach Anspruch 1 oder einem der Ansprüche 2 bis 19, **dadurch gekennzeichnet, dass** im Falle der Inkorporation von Gesamtwasseranteilen > 10 %, bevorzugt ≥ 20 % in die Hartfettpartikel und/oder die niedrigschmelzende, kontinuierliche Öl-/Fettphase in Form disperser Wassertropfen, dieses Fettsystem bei Einsatz als Ziehfett in Blätterteigprodukten bei deren konventionellem Backprozess eine vorteilhafte, kontrollierte Freisetzung des inkorporierten Wasseranteils in Form von Wasserdampf bewirkt, und somit eine deutlich verbesserte Feinstrukturierung des Blätterteigendproduktes erzielen lässt.

21. Fettsystem nach Anspruch 1 oder einem der Ansprüche 2 bis 20, **dadurch gekennzeichnet, dass** im Falle der Inkorporation von Gesamtwasseranteilen ≥ 30 % in die Hartfettpartikel und/oder die niedrigschmelzende, kontinuierliche Öl-/Fettphase in Form disperser Wassertropfen, dieses Fettsystem bei Einsatz als Ziehfett in Blätterteigprodukten bei deren neuartiger Behandlung in einem Mikrowellen-Backprozess eine vorteilhafte, kontrollierte Freisetzung des inkorporierten Wasseranteils in Form von Wasserdampf bewirkt, und somit eine deutlich verbesserte Feinstrukturierung des Blätterteigendproduktes in deutlich reduzierter Backzeit erzielen lässt.

22. Produkt für ein fetthaltiges Lebensmittel mit einem Fettsystem nach Anspruch 1 oder einem der Ansprüche 2 bis 21.

## Claims

1. Edible fat or cosmetic fat system or pharmaceutical fat system with markedly reducedly temperature-dependent consistency and stability characteristics as well as with adjustable, technologically and/or nutritionally relevant functional properties, wherein high-melting, substructured fat particles are suspended in a low-melting fat phase or oil phase or a water/oil emulsion, where by separation into a low- and high-melting oil fraction or hard-fat fraction and arrangement of the high-melting fat phase in the form of suspended, dispersed hard-fat particles in the low-melting oil fraction phase as well as additional substructuring of the dispersed hard-fat particles by means of incorporated, dispersed water drops and/or air bubbles or gas bubbles the temperature-dependency of the viscosity of the entire system over a temperature range of between 10 and 20 degrees Celsius and preferably between 5 and 30 degrees Celsius or even more preferably between 0 and 40 degrees Celsius, quantitatively specified by means of the thermal viscosity coefficient - change of viscosity in kPas per degree of temperature change in degrees Celsius - , is reducedly adjustable by a factor > 3 and preferably by a factor > 5 or > 6.

2. Fat system in accordance with claim 1, **characterised in that** the melting point of the high-melting, dispersed, structured particle phase is > 20°C and the solidification point of the low-melting fat/oil phase < 15°C.

3. Fat system in accordance with any one of claims 1 and 2, **characterised in that** the melting point of the high-melting, dispersed, structured particle phase is > 40°C and the solidification point of the low-melting fat/oil phase < 5°C.

4. Fat system in accordance with any one of claims 1 to 3, **characterised in that** the melting point of the high-melting, dispersed, structured particle phase is > 60°C and the solidification point of the low-melting fat/oil phase < 5°C and preferably < .

5. Fat system in accordance with claim 1 or any one of claims 2 to 4, **characterised in that** the high-melting, dispersed, structured particle fat phase is a solidified emulsion with inner, stabilised water drops and/or the continuous, low-melting fat or oil phase is a water-in-oil emulsion with stabilised water drops.

6. Fat system in accordance with claim 1 or any one of claims 2 to 5, **characterised in that** the high-melting, dispersed particle phase is an oil/water/oil double-emulsion of which the outer oil phase (O) of a high-melting fat with melting temperatures > 20°C is preferably > 40°C, and especially preferably > 60°C and/or the continuous, low-melting fat/oil phase is a wateroil emulsion or an oil-water-oil emulsion, with an outer continuous emulsion oil phase of which the solidification temperatures are < 15°C and preferably < 5°C and even more preferably < 0°C.

7. Fat system in accordance with claim 1 or any one of claims 2 to 6, **characterised in that** the dispersed particle structure in its high-melting fat phase and/or the inner water phase and/or the innermost oil/fat phase incorporates antioxidants and polyunsaturated fatty acids and/or other nutritional or health-promotingly and/or organoleptically relevant functional components.

8. Fat system in accordance with claim 1 or any one of claims 2 to 7, **characterised in that** the continuous fluid phase that corresponds to a low-melting fat/oil system with or without contained emulsion or double-emulsion substructures incorporates antioxidants and polyunsaturated fatty acids and/or other nutritional or health-promotingly and/or organoleptically relevant functional components and/or low-calorie filling components incorporated in a dissolved and/or dispersed form.

9. Fat system in accordance with claim 1 or any one of claims 2 to 8, **characterised in that** nutritional or health-promotingly and/or organoleptically relevant functional components and/or low-calorie filling components are incorporated in a dissolved and/or dispersed form in one or more of the dispersed fat/oil phases and/or water phases.

10. Fat system in accordance with claim 1 or any one of claims 2 to 9, **characterised in that** gas/air bubbles that form a gas-dispersion or foam structure in the respective surrounding phase are incorporated into the continuous fat/oil phase and/or into one or more of the dispersed fat/oil phases and/or water phases.

11. Fat system in accordance with claim 1 or any one of claims 2 to 10, **characterised in that** the mass fraction of the substructured or not substructured, high-melting hard-fat particles in the continuous low-melting oil/fat phase substructured as a water/oil emulsion or a not so substructured oil/fat phase is between 5% and 85% and preferably between 10% and 75% relative to the total mass.

12. Fat system in accordance with claim 1 or any one of claims 2 to 11, **characterised in that** the mass fraction of the dispersed water drops incorporated into the hard-fat particles is between 0% and 80% relative to the hard-fat mass.

13. Fat system in accordance with claim 1 or any one of claims 2 to 12, **characterised in that** the volume fraction of the dispersed gas/air bubblets incorporated into the hard-fat particles is between 0% and 75% relative to the hard-fat volume.

14. Fat system in accordance with claim 1 or any one of claims 2 to 13, **characterised in that** the mass fraction of the dispersed water drops incorporated into the low-melting, continuous oil/fat phase is between 0% and 80% relative to the total mass of the continuous phase.

15. Fat system in accordance with claim 1 or any one of claims 2 to 14, **characterised in that** the flow limit τ_{O,H} of the total fat system determined as a plasticity indicator by means of cone-penetration measurement with maximum deviations of ± 5% from the measured mean value is as independent as possible from the fraction of the water fraction integrated (i) into the hard-fat particles and (ii) into the continuous low-melting oil/fat phase by substructuring in the form of dispersed water drops over a water-content range of between 0% and 80%.

16. Fat system in accordance with claim 1 or any one of claims 2 to 15, **characterised in that** the volume fraction of the dispersed gas/air bubblets incorporated into the low-melting, continuous oil/fat phase is between 0% and 75% relative to the total volume of this continuous phase.

17. Fat system in accordance with claim 1 or any one of claims 2 to 16, **characterised in that** the total fat content is between 20% and 100% and preferably between 50% and 100%.

18. Fat system in accordance with claim 1 or any one of claims 2 to 17, **characterised in that** the total calorie content, when substructuring the dispersed hard-fat particle phase by incorporation of dispersed water drops and/or air/gas bubblets, has been reduced by 50% and preferably by 60%.

19. Fat system in accordance with claim 1 or any one of claims 2 to 18, **characterised in that** the total calorie content, when substructuring the dispersed hard-fat particle phase and the continuous low-melting oil/fat phase by incorporation of dispersed water drops and/or air/gas bubblets, has been reduced by > 50% and preferably by > 70%.

20. Fat system in accordance with claim 1 or any one of claims 2 to 19, **characterised in that,** if total water fractions > 10%, preferably ≥ 20%, are incorporated into the hard-fat particles and/or the low-melting, continuous oil/fat phase in the form of dispersed water drops, this fat system, when used as roll-in fat in layered pastry products during cooking by the conventional baking process, brings about an advantageous, controlled release of the incorporated water fraction in the form of water vapour and so enables a markedly improved fine-structuring of the layered-pastry end product.

21. Fat system in accordance with claim 1 or any one of claims 2 to 20, **characterised in that,** if total water fractions ≥ 30% are incorporated into the hard-fat particles and/or the low-melting, continuous oil/fat phase in the form of dispersed water drops, this fat system, when used as roll-in fat in layered pastry products during cooking by the new method using a microwave baking process, brings about an advantageous, controlled release of the incorporated water fraction in the form of water vapour and so enables a markedly improved fine-structuring of the layered-pastry end product in a markedly reduced baking time.

22. Product for a fat-containing foodstuff using a fat system in accordance with claim 1 or any one of claims 2 to 21.

## Revendications

1. Système de graisse alimentaire ou système de graisse cosmétique ou système de graisse pharmaceutique à comportement de la consistance et de la stabilité nettement moins dépendant de la température, ainsi que doté de propriétés fonctionnelles ajustables, pertinentes au plan technologique et/ou au plan de la physiologie de la nutrition, sachant que des particules de graisse substructurées fondant à haute température sont en suspension dans une phase grasse ou une phase huileuse ou une émulsion eau/huile fondant à basse température, sachant que du fait de la séparation en une fraction d'huile ou fraction de graisse fondant à basse température et en une fraction d'huile ou de graisse fondant à haute température, et du fait que la phase grasse dure fondant à haute température est disposée sous forme de particules de graisse dure en suspension dispersées dans la phase de la fraction d'huile dure fondant à basse température, et du fait de la substructuration supplémentaire - par des gouttes d'eau et/ou par des bulles d'air ou bulles de gaz dispersées incorporées - des particules de graisse dure dispersées, la dépendance en température de la viscosité du système complet peut être ajustée plus faible - d'un facteur > 3, de préférence d'un facteur > 5 ou > 6 - dans une plage de températures comprises entre 10 et 20 degrés Celsius, de préférence entre 5 et 30 degrés Celsius, par ailleurs de préférence entre 0 et 40 degrés Celsius, fait quantitativement décrit par le coefficient de viscosité thermique - modification de la viscosité en kPas par degré Celsius de variation de la température -.

2. Système de graisse selon la revendication 1, **caractérisé en ce que** le point de fusion de la phase particulaire structurée, dispersée, fondant à haute température, est > 20°C, et que le point de solidification de la phase grasse/huileuse fondant à basse température est < 15°C.

3. Système de graisse selon l'une des revendications 1 ou 2, **caractérisé en ce que** le point de fusion de la phase particulaire structurée, dispersée, fondant à haute température, est > 40°C et que le point de solidification de la phase grasse/huileuse fondant à basse température est < 5°C.

4. Système de graisse selon l'une des revendications 1 à 3, **caractérisé en ce que** le point de fusion de la phase particulaire structurée, dispersée, fondant à haute température, est > 60°C et que le point de solidification de la phase grasse/huileuse fondant à basse température est < 5°C, de préférence < 0°C.

5. Système de graisse selon la revendication 1 ou l'une des revendications 2 à 4, **caractérisé en ce que** la phase grasse particulaire structurée, dispersée, fondant à haute température, est une émulsion solidifiée composée de gouttes d'eau stabilisées intérieures et/ou que la phase grasse ou huileuse continue fondant à basse température est une émulsion eau-dans-l'huile composée de gouttes d'eau stabilisées.

6. Système de graisse selon la revendication 1 ou l'une des revendications 2 à 5, **caractérisé en ce que** la phase particulaire dispersée fondant à haute température est une double émulsion huile/eau/huile dont la phase huileuse extérieure (O) est composée d'une graisse fondant à des températures de fusion élevées > 20°C, de préférence > 40°C, en particulier de préférence > 60°C, et/ou **en ce que** la phase grasse/huileuse continue fondant à basse température est une émulsion eau/huile ou une émulsion huile-eau/huile présentant des températures de solidification de < 15°C, de préférence < 5°C, par ailleurs de préférence < 0°C.

7. Système de graisse selon la revendication 1 ou l'une des revendications 2 à 6, **caractérisé en ce que** la structure particulaire dispersée présente, dans sa phase grasse fondant à haute température et/ou dans la phase aqueuse intérieure et/ou dans la phase huileuse/grasse centrale, des antioxydants, des acides gras polyinsaturés et/ou d'autres ingrédients fonctionnels pertinents au plan de la physiologie de la nutrition ou promoteurs de la santé et/ou pertinents au plan organoleptique.

8. Système de graisse selon la revendication 1 ou l'une des revendications 2 à 7, **caractérisé en ce que** la phase fluide continue, qui correspond à un système de graisse/d'huile fondant à basse température avec ou sans substructures d'émulsion et/ou de double émulsion incluses, présente des antioxydants, acides gras polyinsaturés et/ou d'autres ingrédients fonctionnels pertinents au plan de la physiologie de la nutrition ou promoteurs de la santé, et/ou pertinents au plan organoleptique, et/ou des ingrédients de remplissage pauvres en calories incorporés sous une forme dissoute et/ou dispersée.

9. Système de graisse selon la revendication 1 ou l'une des revendications 2 à 8, **caractérisé en ce que** dans une ou plusieurs des phases grasses/huileuses dispersées et/ou des phases aqueuses dispersées sont incorporés des ingrédients fonctionnels pertinents au plan de la physiologie de la nutrition ou promoteurs de la santé et/ou pertinents au plan organoleptique, et/ou des ingrédients de remplissage pauvres en calories incorporés sous une forme dissoute et/ou dispersée.

10. Système de graisse selon la revendication 1 ou l'une des revendications 2 à 9, **caractérisé en ce que** dans la phase grasse/huileuse continue et/ou dans l'une ou plusieurs des phases grasses/huileuses dispersées et/ou des phases aqueuses, des bulles de gaz/d'air ont été incorporées qui créent dans la phase environnante respective une structure à dispersion de gaz ou une structure en mousse.

11. Système de graisse selon la revendication 1 ou l'une des revendications 2 à 10, **caractérisé en ce que** la part massique des particules de graisse dure substructurées ou non substructurées fondant à haute température dans la phase huileuse/grasse continue fondant à basse température, substructurée comme une émulsion eau/huile ou non substructurée de la sorte, représente entre 5 % et 85 %, de préférence entre 10 % et 75 % de la masse totale.

12. Système de graisse selon la revendication 1 ou l'une des revendications 2 à 11, **caractérisé en ce que** la part massique des gouttes d'eau dispersées incorporées dans les particules de graisse dure est comprise entre 0% et 80% de la masse de graisse dure.

13. Système de graisse selon la revendication 1 ou l'une des revendications 2 à 12, **caractérisé en ce que** la part volumique de petites bulles de gaz/d'air dispersées incorporées dans les particules de graisse dure est comprise entre 0% et 75% du volume de graisse dure.

14. Système de graisse selon la revendication 1 ou l'une des revendications 2 à 13, **caractérisé en ce que** la part massique de gouttes d'eau dispersées incorporées dans la phase huileuse/grasse continue fondant à basse température est comprise entre 0% et 80% de la masse totale de la phase continue.

15. Système de graisse selon la revendication 1 ou l'une des revendications 2 à 14, **caractérisé en ce que** la limite d'écoulement τO,H de l'ensemble du système de graisse, déterminée à titre d'indicateur de plasticité par pénétration du cône, dépend très peu, avec des écarts maximaux de l'ordre de ± 5% par rapport à la moyenne mesurée, de la part d'eau intégrée, (i) dans les particules de graisse dure ainsi que (ii) dans la phase huileuse/grasse continue fondant à basse température, par substructuration sous forme de gouttes d'eau dispersées dans une plage de cette même part comprise entre 0% et 80%.

16. Système de graisse selon la revendication 1 ou l'une des revendications 2 à 15 **caractérisé en ce que** la part volumique des petites bulles de gaz/d'air dispersées incorporées dans la phase huileuse/grasse continue fondant à basse température est comprise entre 0% et 75% du volume total de cette phase continue.

17. Système de graisse selon la revendication 1 ou l'une des revendications 2 à 16, **caractérisé en ce que** la teneur totale en graisse est comprise entre 20% et 100%, de préférence entre 50% et 100%.

18. Système de graisse selon la revendication 1 ou l'une des revendications 2 à 17, **caractérisé en ce que** la teneur totale en calorie en cas de substructuration de la phase particulaire dispersée de la graisse dure est réduite de 50%, de préférence de 60%, par incorporation de gouttes d'eau dispersées et/ou de bulles d'air/de gaz dispersées.

19. Système de graisse selon la revendication 1 ou l'une des revendications 2 à 18, **caractérisé en ce que** la teneur totale en calories en cas de substructuration de la phase particulaire dispersée de la graisse dure et de la phase huileuse/grasse continue fondant à basse température est réduite de > 50%, de préférence de > 70%, par incorporation de gouttes d'eau dispersées et/ou de bulles d'air/de gaz dispersées.

20. Système de graisse selon la revendication 1 ou l'une des revendications 2 à 19, **caractérisé en ce que** dans le cas de l'incorporation de parts d'eau totales > 10%, de préférence ≥ 20% dans les particules de graisse dure et/ou dans la phase huileuse/grasse continue fondant à basse température sous forme de gouttes d'eau dispersées, ce système de graisse a pour effet avantageux, lorsque utilisé comme graisse étirée dans les produits à base de pâte feuilletée, lors de leur traitement dans un processus de cuisson conventionel, de libérer de manière contrôlée la part d'eau incorporée sous la forme de vapeur d'eau, et de permettre ainsi d'obtenir une structuration fine nettement meilleure du produit fini à base de pâte feuilletée.

21. Système de graisse selon la revendication 1 ou l'une des revendications 2 à 20, **caractérisé en ce que** dans le cas de l'incorporation de parts d'eau totales > 30% dans les particules de graisse dure et/ou dans la phase huileuse/grasse continue fondant à basse température sous forme de gouttes d'eau dispersées, ce système de graisse a pour effet avantageux, lorsque utilisé comme graisse étirée dans les produits à base de pâte feuilletée, lors de leur traitement nouveau genre dans un processus de cuisson aux micro-ondes, de libérer de manière contrôlée la part d'eau incorporée sous la forme de vapeur d'eau, et de permettre ainsi d'obtenir une structuration fine nettement meilleure du produit fini à base de pâte feuilletée, en un temps de cuisson nettement plus court.

22. Produit pour un produit alimentaire gras contenant un système de graisse selon la revendication 1 ou l'une des revendications 2 à 21.
